# EUROPÄISCHE PATENTSCHRIFT

(11) **EP 1 020 186 B1**
(45) Veröffentlichungstag und Bekanntmachung des Hinweises auf die Patenterteilung: **29.06.2005**
(21) Anmeldenummer: 99125696.7
(22) Anmeldetag: 23.12.1999
(51) Int. Cl.: A61K 31/135, A61K 9/52, A61K 9/54, A61K 9/36, A61K 9/50, A61K 9/62

(54) **Verfahren zur Herstellung einer retardierten Tramadolzubereitung mit einem lagerstabilen Freisetzungsprofil ohne Temperung der gecoateten Zubereitung**
Process for the manufacture of a sustained release tramadol preparation with a storage stable release profile without curing of the coated product
Procédé de fabrication d'une préparation de tramadol à effet retard avec un profil de libération stable au stockage sans recuire la préparation

(30) Priorität: 18.01.1999 DE 19901686
(43) Veröffentlichungstag der Anmeldung: 19.07.2000
(73) Patentinhaber: Grünenthal GmbH, 52078 Aachen (DE)
(72) Erfinder: Bartholomäus, Johannes Heinrich, Dr., 52080 Aachen (DE); Ziegler, Iris, Dr., 52159 Rott-Roetgen (DE)
(74) Vertreter: Kutzenberger, Helga, Dr.

(56) Entgegenhaltungen:
- US-A- 5 645 858
- M. WESSLING ET AL.: "Drug release from beads coated with an aqueous colloidal ethylcellulose dispersion, Aquacoat, or an organic ethylcellulose solution" EUROPEAN JOURNAL OF PHARMACEUTICS AND BIOPHARMACEUTICS , Bd. 47, Nr. 1, Januar 1999 (1999-01), Seiten 33-38, XP000803407 Amsterdam (NL)
- R.U. NESBITT ET AL.: "Effect of substrate on mass release from ethylcellulose latex coated pellets" JOURNAL OF CONTROLLED RELEASE, Bd. 32, Nr. 1, November 1994 (1994-11), Seiten 71-77, XP000476667 Amsterdam (NL)

## Beschreibung

Die vorliegende Erfindung betrifft Verfahren zur Herstellung von vorzugsweise multipartikuläre, orale Tramadolzubereitungen, deren retardiertes Wirkstoff-Freisetzungsprofil durch einen Weichmacher-haltigen Ethylcelluloseüberzug bereits ohne Temperung lagerstabil eingestellt wird.

Aus dem Stande der Technik sind multipartikuläre, retardierte Tramadolzubereitungen mit einem Retardüberzug aus Ethylcellulose bereits bekannt.

So werden in der DE-A-196 30 035 multipartikuläre Tramadolzubereitungen in Form von Pellets, die mit einem Retardüberzug aus Ethylcellulose ausgerüstet sind, beschrieben. Dazu werden die Aufbaupellets mit einer oder mehreren Membranschichten aus Ethylcellulose, die aus Lösungen in organischen Lösungsmitteln aufgebracht werden, überzogen. Diese Herstellung hat den Nachteil, daß die organischen Lösungsmittel aus umweltrelevanten Gründen rückgewonnen werden müssen, was dieses Herstellungsverfahren ziemlich aufwendig macht. Außerdem ist der Einsatz von organischen Lösungsmitteln für die Produktion von Arzneimitteln nachteilig.

Sofern Retardüberzüge aus wässrigen Ethylcellulosedispersionen auf Substrate aufgebracht werden, ist anerkannte Fachmeinung, daß solche Überzüge nach ihrer Herstellung meist keine lagerstabilen Wirkstoff-Freisetzungsprofile gewährleisten. Derartige Ethylcelluloseüberzüge sind nämlich dafür bekannt, daß sie zur sogenannten Nachverfilmung, d. h. einer zunehmenden Verlangsamung der Wirkstofffreisetzung während der Lagerung neigen. Um dieses Problem zu beseitigen und eine lagerstabile Freisetzung des Wirkstoffes zu gewähren, werden nach dem Stand der Technik aufwendige Temperprozesse bei erhöhter Temperatur und ggf. definierter Luftfeuchtigkeit empfohlen, um so innerhalb von Tagen statt einer mehrmonatigen Lagerung ein lagerstabiles Freisetzungsprofil zu erreichen (EP-A-0 548 448, EP-A-0 630 646).

Gemäß der Lehre der US-A-5,645,858 wurde auch vorgeschlagen, eine multipartikuläre Tramadolzubereitung in Form von Aufbaupellets mit mehreren Retardüberzügen aus Ethylcellulose zu versehen, und die so erhaltenen überzogenen Wirkstoffaufbaupellets einem Temperungsprozeß über einen Tag bei erhöhter Temperatur zu unterwerfen, um so ein stabiles Freisetzungsprofil zu erzielen.

Die bekannten Methoden zur Herstellung von Retardüberzügen aus wässriger Ethylcellulosedispersion haben daher den Nachteil, daß erst durch aufwendige Temperungsprozesse oder erst durch einen vielschichtigen Auftrag in Kombination mit einem Temperungsprozeß ein lagerstabiles Wirkstoff-Freisetzungsprofil von Tramadol oder Tramadol-hydrochlorid zielt wird.

Aufgabe der vorliegenden Erfindung war es daher, Verfahren zur Herstellung oraler Tramadolzubereitungen zur Verfügung zu stellen, deren retardierender Ethylcelluloseüberzug trotz Auftrag aus einer wässrigen Ethylcellulosedispersion unmittelbar nach seiner Herstellung ein weitgehend lagerstabiles Wirkstoff-Freisetzungsprofil aufweist.

Erfindungsgemäß gelingt dies durch Bereitstellen des erfindungsgemäßen Verfahrens, gemäß dem eine vorzugsweise multipartikuläre, orale, retardierte Tramadolzubereitung bzw. eine Zubereitung aus einem physiologisch verträglichen Salz von Tramadol mit einem lagerstabilen Wirkstoff-Freisetzungsprofil hergestellt wird. Dazu wird die vorzugsweise multipartikuläre Wirkstoffzubereitung mit einer wässrigen Ethylcellulosedispersion überzogen, die mindestens einen physiologisch verträglichen, lipophilen Diester aus einer aliphatischen oder aromatischen Dicarbonsäure mit C₆-C₄₀ und einem aliphatischen Alkohol mit C₁-C₈ als Weichmacher enthält, und der Überzug gleichzeitig bei Produkttemperaturen von 35°C bis 80°C trocknet, ohne dass eine an die Trocknung anschließende Temperung durchgeführt wird.

Überraschenderweise zeigen so hergestellte retardierte Tramadolzubereitungen bereits unmittelbar nach ihrer Herstellung ein lagerstabiles Wirkstoff-Freisetzurrgsprofil, ohne daß eine an die übliche Trocknung anschlie-ßende Temperung notwendig wäre. Die erfindungsgemäß hergestellten, retardierten Tramadolzubereitungen weisen einen sogenannten koaleszierten Ethylcelluloseüberzug auf, bei dem die diskreten Ethylcellulosepartikel zu einem Überzug zusammengeflossen sind.

Tramadol oder vorzugsweise ein physiologisch verträgliches Salz von Tramadol, wie Tramadolhydrochlorid, wird für das erfindungsgemäße Verfahren in Form von Tabletten, Mikrotabletten, Granulaten, Kristallen, Pellets, wie Extrusionspellets oder Aufbaupellets vorzugsweise mit einer Größe von 0,3 bis 2,5 mm eingesetzt. Die Herstellung solcher multipartikulären Substraten ist dem Fachmann bekannt.

Zur Herstellung der Retardüberzüge werden wässrige Ethylcellulosedispersionen eingesetzt, die eine Konzentration von 3 bis 35 Gew.%, vorzugsweise von 10 bis 25 Gew.%, wasserunlöslicher Ethylcellulose aufweisen. Die wässrigen Ethylcellulosedispersionen, die als Überzugsmaterial zum Einsatz kommen, enthalten mindestens einen physiologisch verträglichen lipophilen Diester aus einer aliphatischen oder aromatischen Dicarbonsäure mit C₆-C₄₀, vorzugsweise C₆-C₃₀, besonders bevorzugt C₁₀-C₁₆, und einen aliphatischen Alkohol mit C₁-C₈, vorzugsweise C₂-C₆, besonders bevorzugt C₂-C₅, als Weichmacher. Bevorzugt werden als Weichmacher Dibutylphtalat, Diethylphtalat, Dibutylsebacat oder Diethylsebacat, besonders bevorzugt Dibutylsebacat, eingesetzt. Die Menge des Weichmachers beträgt 5 bis 50 Gew.%, vorzugsweise 10 bis 40 Gew.%, besonders bevorzugt 10 bis 30 Gew.%, bezogen auf Ethylcellulose. Ganz besonders bevorzugt sind Retardüberzüge aus Ethylcellulose, die 10 bis 30 Gew.%, bezogen auf Ethylcellulose, Dibutylsebacat enthalten.

Die zum Einsatz kommenden, wässrigen Ethylcellulosedispersionen können, Handelsprodukte, wie z. B. Aquacoat™ oder Surelease™ sein. Dabei können diese wie z.B. Surelease bereits den notwendigen Weichmacher enthalten. Es ist aber auch möglich, die Weichmacher in die wässrige Ethylcellulosedispersion vorzugsweise mit Hilfe von Tensiden oder Emulgatoren, wie z. B. Polysorbat 80 (Tween 80®), einzuarbeiten. Ganz besonders bevorzugt wird als wässrige Ethylcellulosedispersion eine Dispersion eingesetzt, die bereits den Weichmacher während der Herstellung der Ethylcellulosedispersion als Komponente enthält, wie das Handelsprodukt Surelease E-7-7050™.

Die unmittelbar nach der Herstellung erhaltenen Freisetzungsprofile können durch die dem Fachmann bekannten Methoden, wie z. B. durch die jeweilige Dicke des Überzugs oder den Zusatz von weiteren Hilfsstoffen als Bestandteile des Überzugs eingestellt werden. Pigmente, wie Eisenoxide, Titandioxid, Gleitmittel, wie Talkum, Aerosil, Glycerinmonostearat, hydrophile Porenbildner wie Lactose, Polyethylenglycol, Mannitol und/oder wasserlösliche Polymere, wie Hydroxypropylmethylcellulose, Polyvidon kommen dafür in Frage. Es ist auch möglich, durch Mischen mit anderen Überzugsdispersionen, wie z. B. Eudragit™, RS 30D, RL 30D, NE 30D, Dispersionen magensaftresistenter Filmbildner, wie z. B. Endragit L 30D, die Freisetzung des Wirkstoffes so zu steuern, daß eine Retardierungen im Bereich von mindestens 4 Stunden bis zu 24 Stunden erreicht wird.

Der retardierende Überzug basierend auf Ethylcellulose wird hergestellt, indem man die Wirkstoffsubstrate nach ihrer Herstellung mit der wässrigen Dispersion durch Aufsprühen, vorzugsweise mit Hilfe des Wirbelschichtverfahrens überzieht und gleichzeitig bei üblichen Temperaturen trocknet. Vorzugsweise werden dabei Produkttemperaturen von mindestens 35°C, bevorzugt 35°C bis 80°C, besonders bevorzugt von 40°C bis 45°C, angestrebt, die mit Hilfe von Zuluft mit Temperaturen von mindestens 50°C, bevorzugt 55°C bis 70°C eingestellt werden.

Um das sehr gut wasserlösliche Tramadol bzw. Tramadolsalz, wie Tramadolhydrochlorid, beim Überziehen mit der wässrigen Ethylcellulosedispersion zu schützen, kann es von Vorteil sein, über das Wirkstoffsubstrat vor dem Aufbringen des Retardüberzugs einen Schutzüberzug aufzubringen. Vorzugsweise wird der Schutzüberzug in einer Menge von 1 bis 10 Gew.%, vorzugsweise 2,5 bis 5 Gew.%, bezogen auf die Megen des zu überziehenden Wirkstoffsubstrates, zur Isolation aufgetragen.

Dies führt zu keiner wesentlichen Reduktion des für die Retardierung benötigten Auftrags, vermeidet aber das Auflösen des wasserlöslichen Wirkstoffes während des Überziehens mit Ethylcellulose und verhindert ein Eindringen des Wirkstoffes in den Retardfilm.

Wässrige Lösungen von wasserlöslichen Polymeren, wie z. B. Hydroxypropylmethylcellulose oder Hydroxypropylcellulose, Polyvidon mit oder ohne weitere Hilfsstoffe, wie z. B. Talkum oder durch Schmelzüberziehen mit oder aus organischen Lösungen aufgebrachte lipophile Substanzen, wie z. B. Fettalkohole, Fettsäuren, Fette, Wachse, Glycerolmonostearat, Glycerolbehenat Typen können als Schutzüberzugsmaterial verwendet werden.

Außenüberzüge über die retardierten Substrate werden in der Regel aufgebracht, um das Verkleben der Substrate während der Lagerung zu verhindern. Außenüberzüge können aber auch funktionelle Überzüge sein, die einen zusätzlichen Schutz vor z. B. erhöhter Luftfeuchte oder vor dem Eindringen von Magensäure in das Substrat verhindern. Die Menge des Außenüberzugs richtet sich dabei nach seiner Funktion und reicht von ≤ 1 Gew.% als Verklebungsschutz bis zu 30 Gew.% zur Erhöhung der Magensaftresistenz.

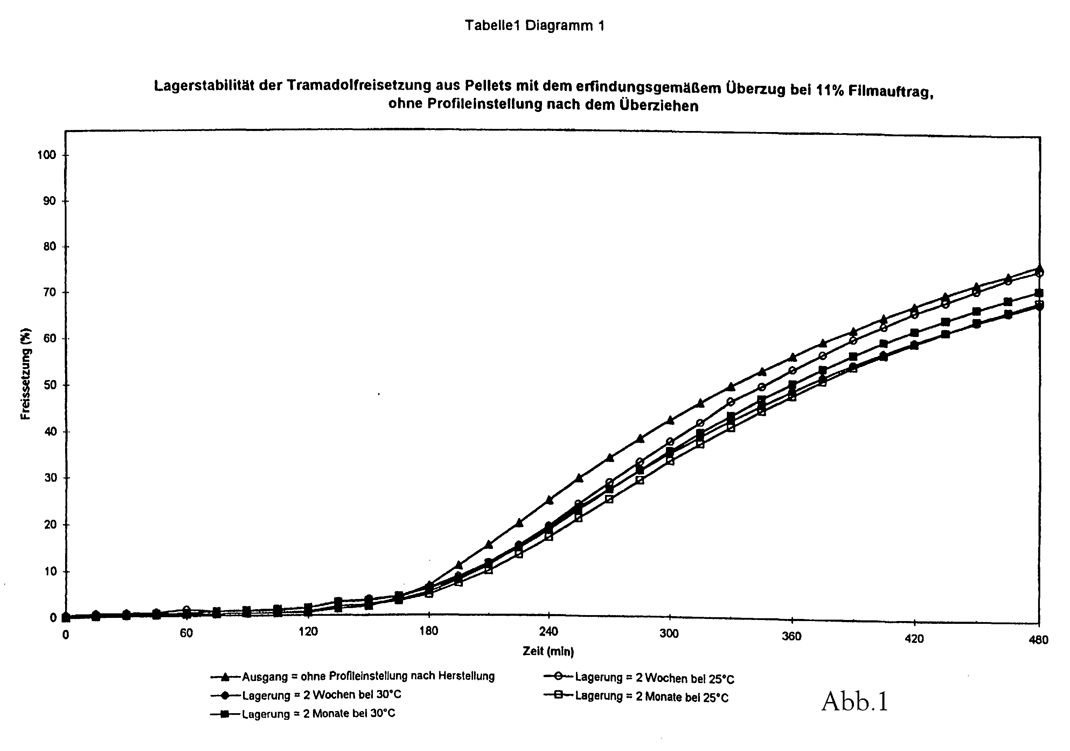

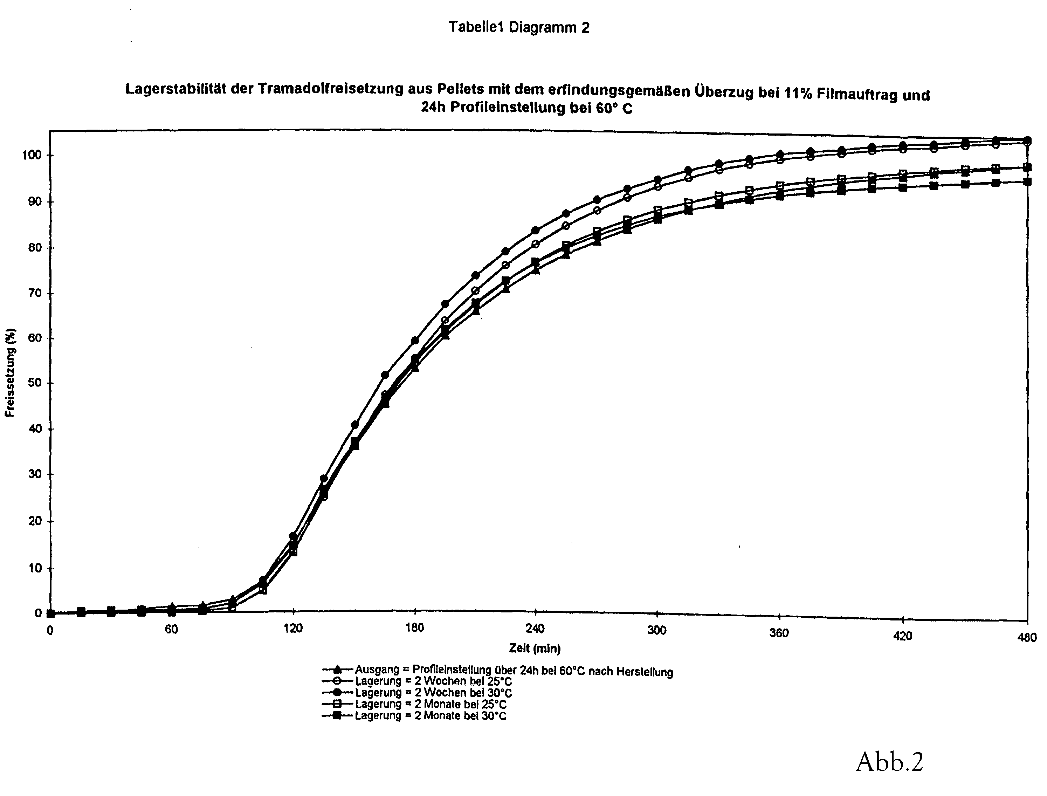

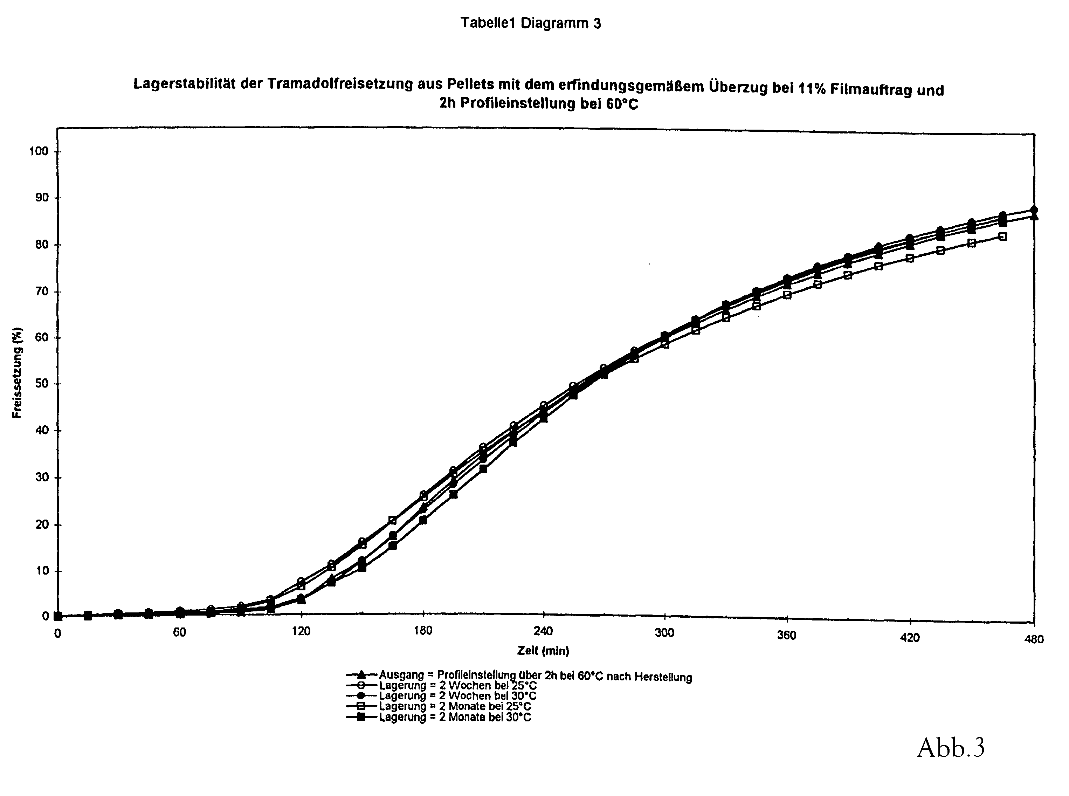

Wie Abb. 1 - Abb. 3 zeigen, bleiben alle drei Freisetzungsprofile für Tramadol-HCI bei den üblichen Lagerbedingungen von 25°C bis 30°C über die gesamte Lagerzeit hinweg und bei beiden Lagertemperaturen unverändert.

Die Lagerstabilität nach USP 23, Seiten 1959 ff "[1196] the Stability Testing of new drug stubstances and products - The tripartite Guideline" gemessen.

Dabei zeigen sie trotz identischer Filmschichtdicke und Filmzusammensetzung im kombinierten Dissolutiontest aus 2 h Magensaft pH 1,2 und 8 h Darmsaft pH 7,2 von einander völlig verschiedene Ausgangsfreisetzungen. Während der Film ohne zusätzliche Behandlung nach der Herstellung die stärkste Retardierung mit einer Freisetzung von ca. 75 % in 8 h zeigt, setzt der gleiche Film nach 24 h Tempern bei 60°C bereits 100 % nach 5 h frei. Eine Tempern von 2 h bei 60°C resultiert in einer Tramadolfreisetzung von ca. 90 % in 8 h. Diese unterschiedlichen Freisetzungsprofile zeigen, daß durch Überzüge aus koaleszierter Ethylcellulose mit den entsprechenden Weichmachern eine bedarfsgerechte lagerstabile Tramadolfreisetzung möglich wird, die je nach Bedarf sowohl unmittelbar nach Herstellung erfolgen kann oder auch erst nach Zwischenlagerung der mit dem erfindungsgemäßen Überzug überzogenen Tramadolzubereitungen. Dadurch ergibt sich für die großtechnische Produktion der Zubereitungen der große Vorteil einer jederzeit möglichen Aufarbeitung von Chargen mit zu langsamen Freisetzungsprofil.

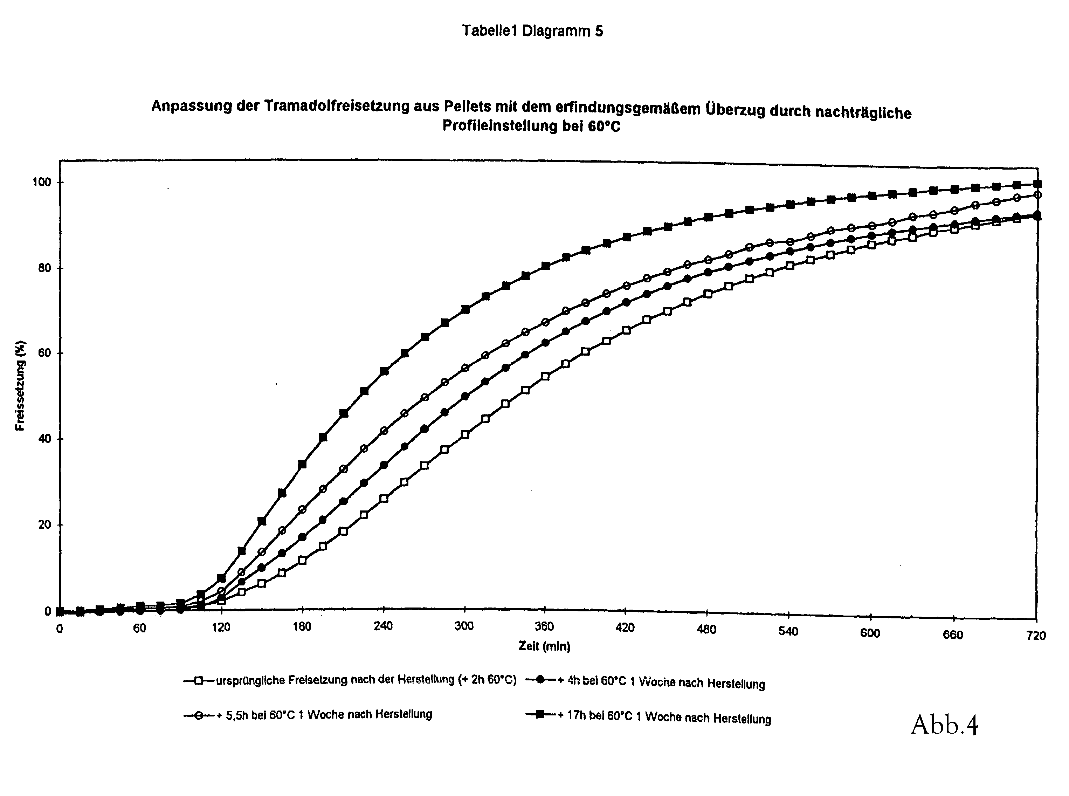

Abb. 4 zeigt, wie das Freisetzungsprofil von Tramadol HCI-Pellets mit dem erfindungsgemäßen Überzug und einer unmittelbar nach der Herstellung relativ langsamen Freisetzung von kleiner 45 % nach 300 min durch nachträgliches Erhitzen schrittweise in Richtung einer schnelleren Freisetzung, bis hin zu 70 % in 300 min verändert werden kann.

Die erfindungsgemäßen, retardierten Tramadol HCI-Zubereitungen wurden neben dem Standarddissolutiontest von 2 Stunden Magensaft + 6 Stunden Darmsaft zusätzlich auch 8 Stunden mit pH-Gradient von pH 1,2 bis pH 7,2, 8 Stunden in künstlichem Darmsaft pH 7,2 mit 100 mM NaCl (250 mM KH₂PO₄ + 100 mM NaCl), 8 Stunden in künstlichem Darmsaft pH 6,8 (220 mM KCl + 30 mM KH₂PO₄), 8 Stunden in künstlichem Magensaft pH 1,2, 8 h in Puffer pH 4,6 (100 mM NaC₂H₃O₂ + 50 mM NaCl) und 8 Stunden in künstlichem Darmsaft pH 6,8 mit 5 mM Na-Taurocholat (220 mM KCI + 30 mM KH₂PO₄) geprüft. Wenn nicht anders angegeben wurde die Freisetzung im Körbchen bei einer Rotationsgeschwindigkeit von 100 min⁻¹ geprüft. Es wurden aber auch alternativ andere Rotationsgeschwindigkeiten untersucht, um den Einfluß der mechanischen Belastung auf die Freisetzung zu sehen.

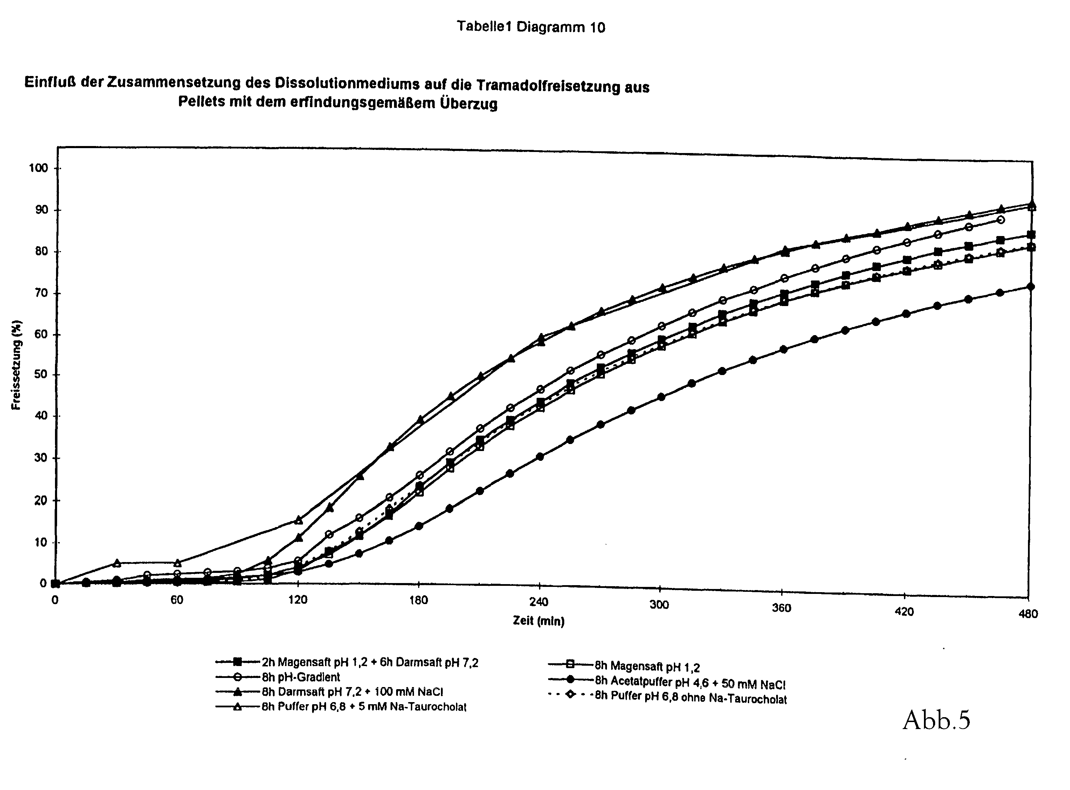

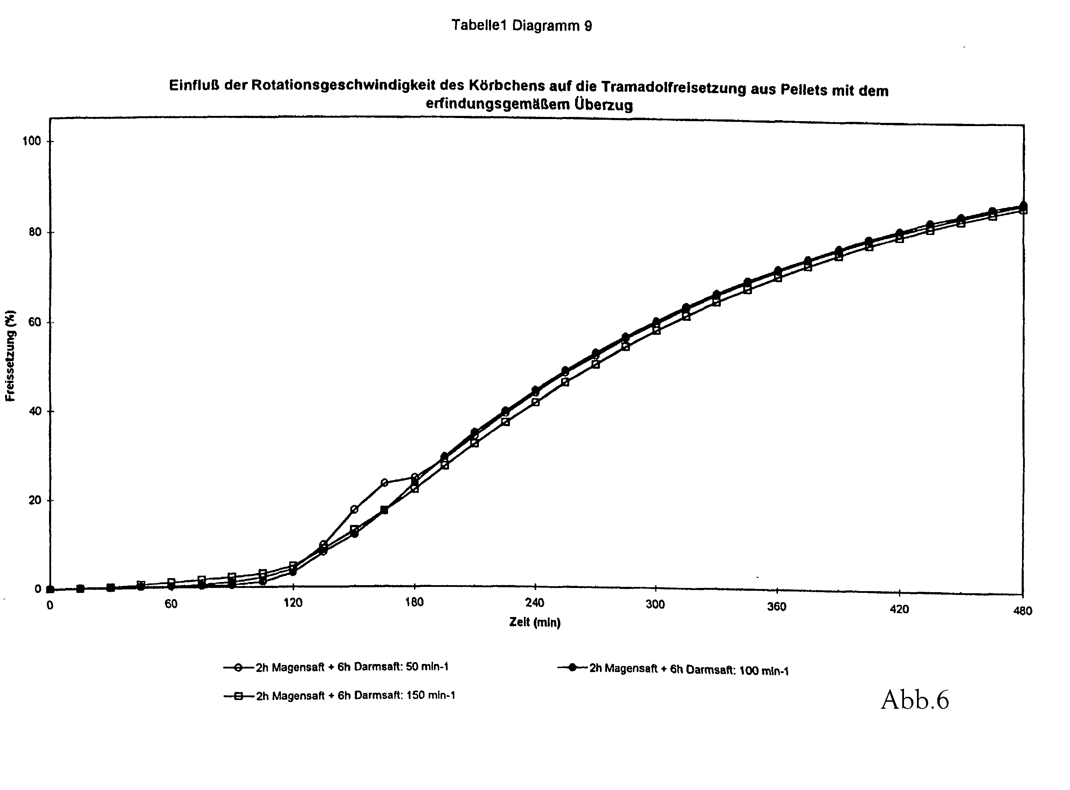

Wie aus Abb. 5 und Abb. 6 ersichtlich, zeigten weder die Zusammensetzung des Freisetzungsmediums hinsichtlich Molarität, pH-Wert oder lonenart noch das Ausmaß der mechanischen Belastung der Pellets einen großen Einfluß auf die Tramadolfreisetzung aus mit dem erfindungsgemäßen Überzug überzogenen Pellets. Dadurch bestätigt sich ein robustes Freisetzungsverhalten der erfindungsgemäßen Zubereitungen hinsichtlich der in-vitro Testung, so daß auch in-vivo eine zuverlässige Freisetzung zu erwarten ist.

### Beispiele

Die in -vitro Freisetzung von Tramadol wurde im Dissolutiontest nach Ph. Eur. mit der Körbchenmethode bei einer Rotationsgeschwindigkeit von 100 min⁻¹ bestimmt. Wenn nicht anders beschrieben wurde die Zubereitung zuerst 2 Stunden lang in künstlichem Magensaft pH 1,2 und anschließend weitere 6 Stunden in künstlichem Darmsaft pH 7,2 geprüft. Die jeweils zum Meßzeitpunkt in Lösung befindliche Menge an Tramadol wurde spektralphotometrisch bestimmt und in Prozent der Gesamtdosis an Tramadolhydrochlorid angegeben. Die angegebenen Freisetzungswerte und Kurven entsprechen dem Mittelwert aus n = 3.

### Beispiel 1

Tramadol HCI-Pellets mit einem Wirkstoffgehalt von 70 Gew.% wurden durch wässrige Granulation mit Mikrokristalliner Cellulose und niedrig substituierter Hydroxypropylcellulose, Extrusion und anschließender Spheronisation hergestellt. Die getrockneten Pellets mit einer ausgesiebten Größe von 800- 1250 pm wurden dann nach dem Wirbelschichtverfahren bei 60°C Zulufttemperatur zuerst mit 3 Gew.% Schutzüberzug aus Hydroxypropylmethylcellulose, PEG 400 und Talkum überzogen und dann mit 11 Gew.%, bezogen auf das Gewicht der mit Schutzüberzug versehenen Pellets, mit einem Retardüberzug versehen.

Die Zusammensetzung der wässrigen Dispersion zur Herstellung eines Schutzüberzuges auf 5 kg Pellets

| | |
|---|---|
| Hydroxypropylmethylcellulose (Pharmacoat 603/ShinEtsu) | 104,0 g |
| PEG 400 | 12,0 g |
| Talkum mikronisiert | 35,0 g |
| Ger. Wasser | 2160,0 g |
| Gesamt: | 2311,0 g |

Zusammensetzung der wässrigen Überzugsdispersion zum Überziehen von 5 kg mit Schutzüberzug versehenen Pellets

| | |
|---|---|
| Surelease E-7-7050 (wäßrige Ethylcellulosedispersion:Coloron | 2115,0 g |
| Ger. Wasser | 1323,0 g |
| Gesamt: | 3438,0 g |

Feststoffgehalt der Dispersion = 16 Gew.%

Nach dem Überziehen der Pellets erfolgte entweder kein Tempern oder eine Wirkstoff-Freisetzungeinstellung für 2 Stunden bei 60°C.

163 mg Pellets, entsprechend einer Dosis von 100 mg Tramadolhydrochlorid, wurden in Kapseln der Größe 1 gefüllt und die Wirkstofffreisetzung wie oben angegeben ermittelt.

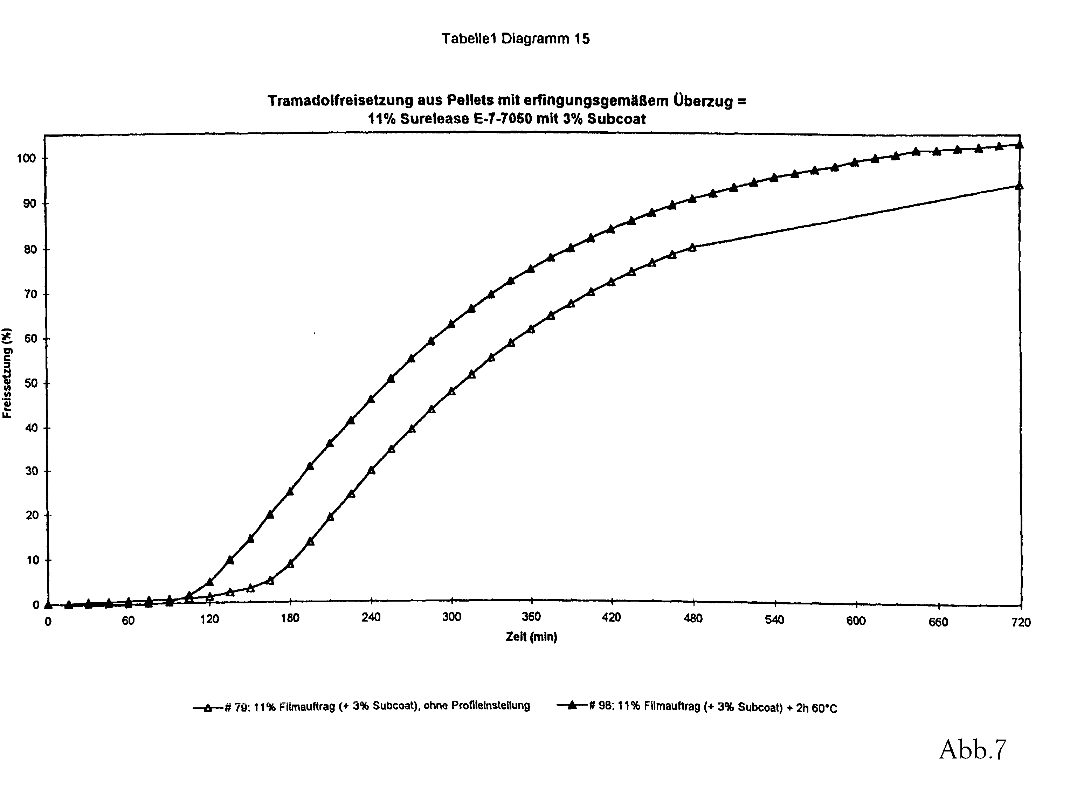

Die angegebenen Freisetzungswerte entsprechen dem Mittelwert aus n = 6. (Abb. 7)

| Zeit in min. | Freigesetzter Anteil in % | Freigesetzter Anteil in % nach 6 Monaten Lagerung bei 25°C | Freigesetzter Anteil in % nach 6 Monaten Lagerung bei 30°C | Freigesetzter Anteil in % nach Behandlung für 2 h bei 60°C | Freigesetzter Anteil in % nach 6 Monaten Lagerung bei 25°C | Freigesetzter Anteil in % nach 6 Monaten Lagerung bei 30°C |
|---|---|---|---|---|---|---|
| 120 | 1 | 1 | 1 | 5 | 4 | 5 |
| 240 | 29 | 26 | 27 | 46 | 45 | 48 |
| 360 | 61 | 61 | 60 | 75 | 70 | 74 |
| 480 | 80 | 79 | 78 | 91 | 86 | 90 |
| 600 | 94 | 95 | 94 | 99 | 98 | 100 |

### Referenzbeispiel 2

Tramadol HCI-Pellets mit einem Wirkstoffgehalt von 55 Gew.% wurden durch wässrige Granulation mit mikrokristalliner Cellulose und niedrig substituierter Hydroxypropylcellulose, Extrusion und anschließender Spheronisation hergestellt. Die getrockneten Pellets mit einer ausgesiebten Größe von 800 - 1250 µm wurden dann nach dem Wirbelschichtverfahren bei 60°C Zulufttemperatur mit 8 Gew.% Gesamtüberzugsauftrag, bezogen auf Ausgangsgewicht der Pellets, überzogen.

Zusammensetzung der wässrigen Überzugsdispersion zum Überziehen von 300 g Pellets

| | |
|---|---|
| Aquacoat ECD 30 (wäßrige Ethylcellulosedispersion) | 53,0 g |
| Dibutylsebacat | 4,8 g |
| Talkum (mikronisiert) | 3,2 g |
| Polysorbat 80 | 0,02 g |
| Siliciumemulsion | 0,02 g |
| Gereinigtes Wasser | 65,0 g |
| Gesamt: | 126,0 g |

Feststoffgehalt der Dispersion = 19 Gew.%

Nach dem Überziehen der Pellets und Trocknen des Überzugs erfolgte eine Wirkstofffreisetzungs-Einstellung 60°C für 2 bzw. 27 Stunden.

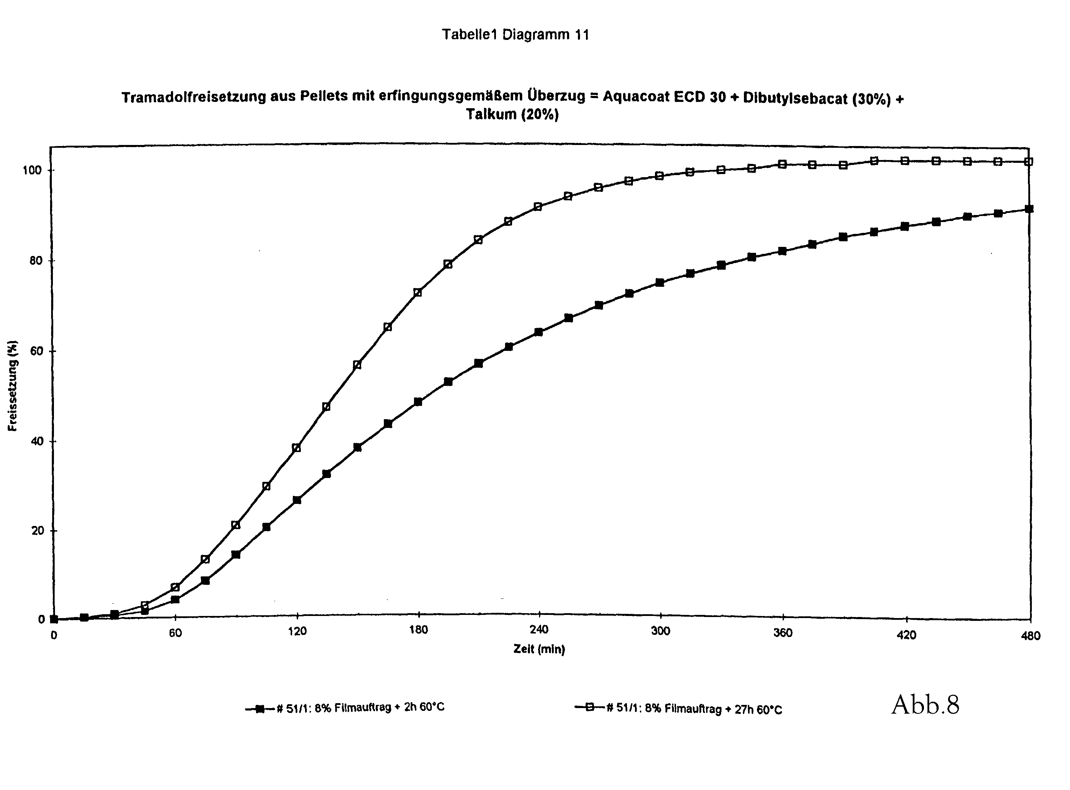

196 mg dieser Pellets, entsprechend einer Dosis von 100 mg Tramadolhydrochlorid, werden in Kapseln der Größe 1 abgefüllt und die Wirkstofffreisetzung wie oben angegeben bestimmt. Die nachstehend angegebenen Freisetzungswerte entsprachen dem Mittelwert aus n = 3. (Abb. 8)

| Zeit in min. | Freigesetzter Anteil in % Behandlung 2 h 60°C | Freigesetzer Anteil in % Behandlung 27 h 60°C |
|---|---|---|
| 120 | 26 | 38 |
| 240 | 63 | 91 |
| 360 | 82 | 101 |
| 480 | 92 | 101 |

### Referenzbeispiel 3

Tramadol HCI-Pellets mit einem Wirkstoffgehalt von 55 Gew.% wurden durch wässrige Granulation mit mikrokristalliner Cellulose und niedrig substituierter Hydroxypropylcellulose, Extrusion und anschließender Spheronisation hergestellt. Die getrockneten Pellets mit einer ausgesiebten Größe von 800 - 1250 µm wurden dann nach dem Wirbelschichtverfahren bei 60°C Zulufttemperatur zuerst mit 0,6 Gew.% eines Schutzüberzugs und dann mit 15 Gew.% Gesamtüberzugsauftrag, bezogen auf die mit Schutzüberzug vesehenen Pellets, überzogen.

Zusammensetzung der wässrigen Überzugsdispersion zur Herstellung eines Schutzüberzuges für 350 g Pellets

| | |
|---|---|
| Opadry OY-29020 clear (=Hydroxypropylmethylcellulose und PEG 400: Coloron) | 1,60 g |
| Talkum mikronisiert | 0,50 g |
| Ger. Wasser | 27,9 g |
| Gesamt: | 30,0 g |

Zusammensetzung der wässrigen Überzugsdispersion zum Überziehen von 300 g mit Schutzüberzug versehenen Pellets

| | |
|---|---|
| Aquacoat ECD 30 (wäßrige Ethylcellulosedispersion: Colorcon FMC) | 89,0 g |
| Opadry OY-29020 clear | 3,0 g |
| | |
| Dibutylsebacat | 7,6 g |
| Talkum (mikronisiert) | 7,7 g |
| Polysorbat 80 | 0,03 g |
| Siliciumemulsion | 0,03 g |
| Ger. Wasser | 129,6 g |
| Gesamt: | 237,0 g |

Feststoffgehalt der wässrigen Dispersion = 19 Gew.%

Nach der Herstellung der mit Retardüberzug versehenen Pellets erfolgte die Profileinstellung durch Tempern bei 60°C für 2 Stunden. 210 mg Pellets, entsprechend einer Dosis von 100 mg Tramadolhydrochlorid, wurden mit 192, 1 mg Cellactose, 16,8 mg Kollidon CL® (=Crospovidone) und 1,1 mg Magnesiumstearat zu Tabletten mit 12 mm Durchmesser und 420 mg Gewicht verpreßt. Diese zerfielen in Wasser innerhalb von 1 - 2 Minuten wieder in die einzelnen Pellets. Die Bestimmung der Wirkstofffreisetzung erfolgte wie oben angegeben.

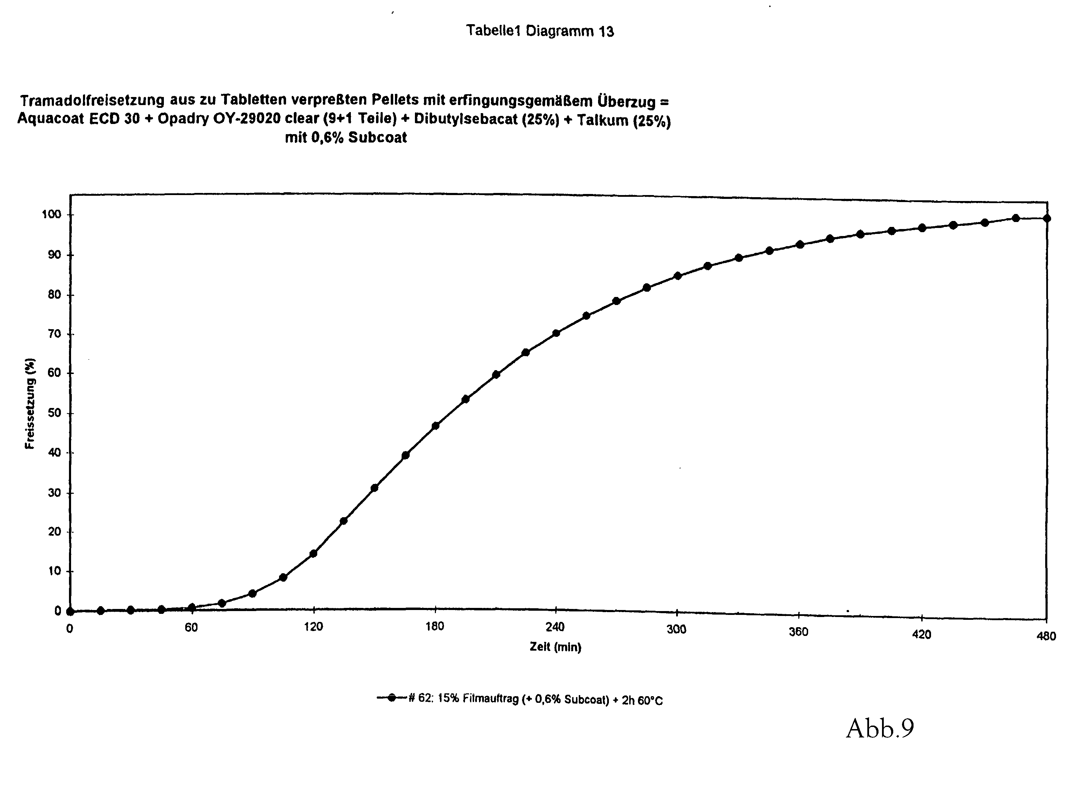

Die nachstehend angegebenen Freisetzungswerte entsprachen dem Mittelwert aus n = 3. (Abb. 9)

| Zeit in min. | Freigesetzer Anteil in % (Behandlung bei 60°C, 2h) |
|---|---|
| 120 | 14 |
| 240 | 70 |
| 360 | 94 |
| 480 | 101 |

### Referenzbeispiel 4

Tramadolhydrochlorid-Pellets mit einem Wirkstoffgehalt von 33 Gew.% wurden durch wässrige Granulation mit mikrokristalliner Cellulose und niedrig substituierter Hydroxypropylcellulose, anschließender Extrusion und Spheronisation hergestellt. Die getrockneten Pellets mit einer ausgesiebten Größe von 800 - 1250 µm wurden dann nach dem Wirbelschichtverfahren bei 60°C Zulufttemperatur mit 6 Gew.% Gesamtfilmauftrag, bezogen auf das Gewicht der nicht überzogenen Pellets, überzogen.

Zusammensetzung der wässrigen Überzugsdispersion zum Überziehen von 350 g Pellets

| | |
|---|---|
| Aquacoat ECD 30 | 58,3 |
| (wäßrige Ethylcellulosedispersion: Colorcon) Dibutylsebacat | 3,5 g |
| Polysorbat 80 (Tween 80) | 0,01 g |
| wässrige Siliconemulsion | 0,01 g |
| gereinigtes Wasser | 78,2 g |
| (ger. Wasser) | |
| Gesamt: | 140,0 g |

Als Antischaumregulator wurde in allen Beispielen eine wässrige Siliconemulsion verwendet.
Feststoffgehalt der Dispersion = 15 Gew.%

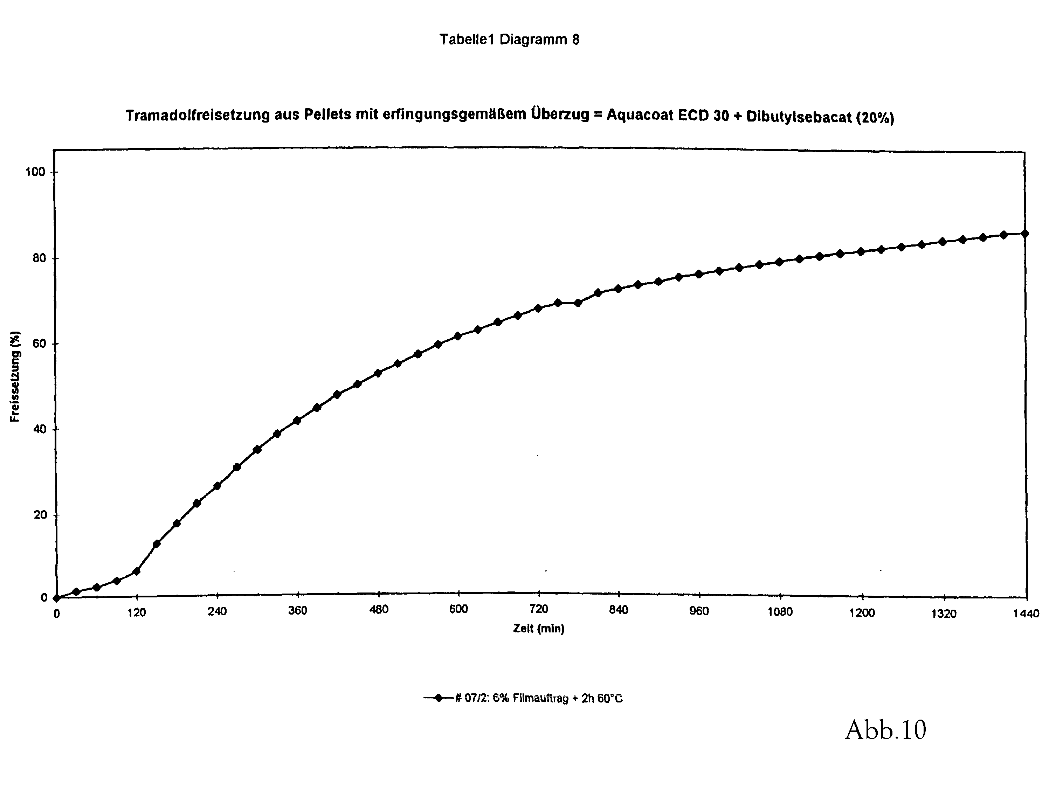

Nach dem Überziehen und Trocknen wurden die Pellets bei 120°C für 60 min gehalten. 321 mg Pellets, entsprechend einer Dosis von 100 mg Tramadolhydrochlorid wurden in Kapseln der Größe 0 abgefüllt und die Wirkstofffreisetzung daraus bestimmt. (Abb. 10)

| Zeit in min. | Freigesetzter Anteil in % |
|---|---|
| 120 | 23 |
| 360 | 36 |
| 600 | 55 |
| 990 | 72 |
| 1440 | 83 |

### Beispiel 5

Es wurden Tabletten mit 10 mm Durchmesser und der folgenden Zusammensetzung auf einer Tablettenpresse hergestellt:

| | |
|---|---|
| Tramadolhydrochlorid | 100,0 mg |
| Mikrokristalline Cellulose (Avicel PH 101) | 80,0 mg |
| Polyvidon K30 | 16,0 mg |
| Magnesiumstearat | 4,0 mg |
| Gesamt: | 300,0 mg |

Tramadolhydrochlorid und Mikrokristalline Cellulose wurden mit einer wässrigen Lösung von Polyvidon K30 granuliert, getrocknet, gesiebt und nach Abmischen mit Magnesiumstearat zu Tabletten mit einem Gewicht von 300 mg verpreßt.

Die Tabletten wurden im Trommel-Coater bei 60°C Zulufttemperatur mit 5 Gew.% Ethylcellulose Retardfilm (bezogen auf das Gewicht der Tabletten) überzogen, so daß sich ein Tablettengewicht von 315 mg ergibt.

Zusammensetzung der Überzugdispersion zum Überziehen von 600 g Tabletten.

| | |
|---|---|
| Surelease E-7-7050 wäßrige Ethylcellulosedispersion: Colorcon | 115,4 g |
| Ger. Wasser | 72,1 g |
| Gesamt: | 187,5 g |

Feststoffgehalt der Dispersion = 16 Gew.%

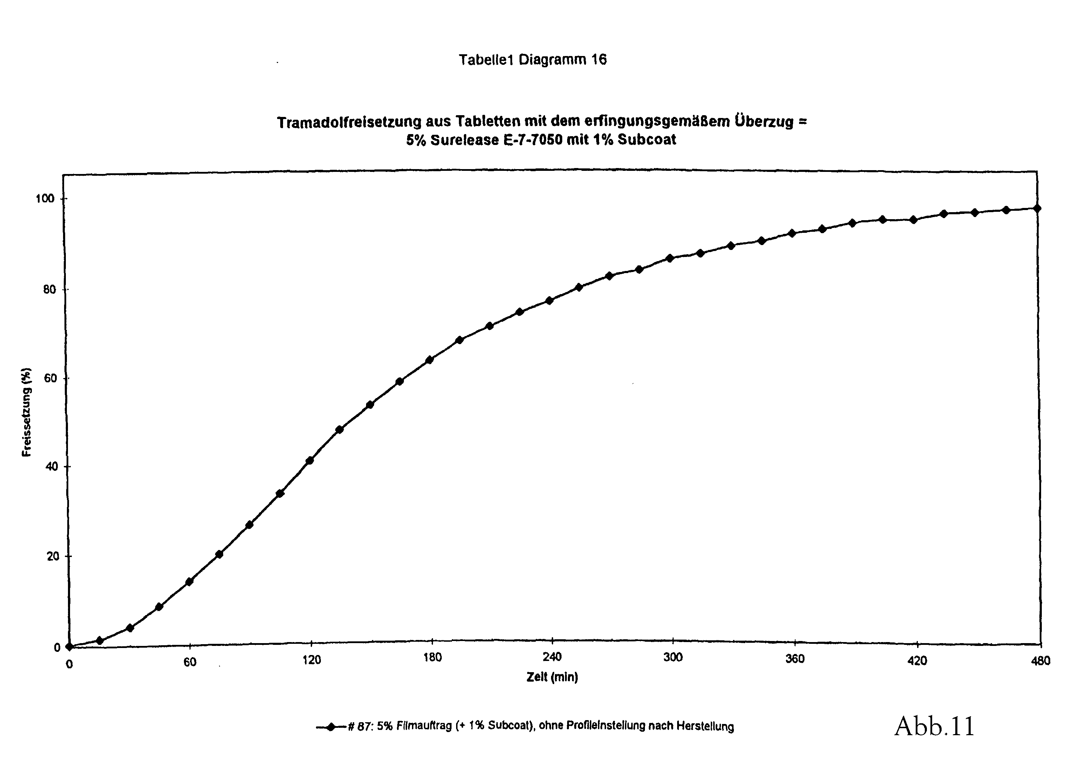

Nach dem Überziehen der Tabletten erfolgte kein Tempern. Die Wirkstofffreisetzung wird wie oben angegeben bestimmt, wobei die angegebenen Freisetzungswerte dem Mittelwert aus n = 2 entsprechen. (Abb. 11)

| | |
|---|---|
| Zeit in min. | Freigesetzer Anteil in % |
| 120 | 40 |
| 240 | 76 |
| 360 | 91 |
| 480 | 97 |

## Patentansprüche

1. Verfahren zur Herstellung einer oralen, retardierten Zubereitung aus Tramadol oder einem physiologisch verträglichen Salz von Tramadol mit einem lagerstabilen Wirkstoff-Freisetzungsprofil, **dadurch gekennzeichnet, daß** man die Wirkstoffzubereitung mit einer wässrigen Ethylcellulosedispersion überzieht, die wenigstens einen physiologisch verträglichen, lipophilen Diester aus einer aliphatischen oder aromatischen Dicarbonsäure mit C₆-C₄₀ und einem aliphatischen Alkohol mit C₁-C₈ als Weichmacher enthält, und den Überzug bei Produkttemperaturen von 35°C bis 80°C nur während des Überziehens trocknet ohne eine an die Trocknung anschließende Temperung.

2. Verfahren nach Anspruch 1, **dadurch gekennzeichnet, daß** als Weichmacher ein Diester aus einer aliphatischen oder aromatischen Dicarbonsäure mit C₆-C₃₀, bevorzugt C₁₀-C₁₆, und einem aliphatischen Alkohol mit C₂-C₆, bevorzugt C₂-C₅, eingesetzt wird.

3. Verfahren nach Anspruch 2, **dadurch gekennzeichnet, daß** als Weichmacher Dibutylphtalat, Diethylphtalat, Dibutylsebacat oder Diethylsebacat, vorzugsweise Dibutylsebacat, eingesetzt wird.

4. Verfahren nach einem der Ansprüche 1 bis 3, **dadurch gekennzeichnet, daß** der Weichmacher in Mengen von 5 bis 50 Gew.%, vorzugsweise 10 bis 40 Gew.%, besonders bevorzugt 10 bis 30 Gew.%, bezogen auf Ethylcellulose, eingesetzt wird.

5. Verfahren nach einem der Ansprüche 1 bis 4, **dadurch gekennzeichnet, daß** die Konzentration der Ethylcellulose in der wässrigen Dispersion von 3 Gew.% bis 35 Gew.%, vorzugsweise 10 bis 30 Gew.% beträgt.

6. Verfahren nach einem der Ansprüche 1 bis 5, **dadurch gekennzeichnet, daß** die Trocknung des retardierenden Überzugs bei Temperaturen von 40 bis 80°C, vorzugsweise 50 bis 70°C, erfolgt.

7. Verfahren nach einem der Ansprüche 1 bis 6, **dadurch gekennzeichnet, daß** unter dem retardierenden Überzug ein Schutzüberzug aus wasserlöslichen Polymeren, vorzugsweise Hydroxypropylmethylcellulose oder Hydroxypropylcellulose und Talkum aufgetragen wird.

8. Verfahren nach Anspruch 7, **dadurch gekennzeichnet, daß** die Gewichtszunahme durch den Schutzüberzug 1 bis 10 Gew.%, vorzugsweise 2,5 bis 5 Gew.%, beträgt.

9. Verfahren nach einem der Ansprüche 1 bis 8, **dadurch gekennzeichnet, daß** das physiologisch verträgliche Salz von Tramadol Tramadolhydrochlorid ist.

10. Verfahren nach einem der Ansprüche 1 bis 9, **dadurch gekennzeichnet, daß** die Wirkstoffzubereitung als multipartikuläre Wirkstoffzubereitung, vorzugsweise als Tabletten, Mikrotabletten, Granulate, Pellets oder Kristalle eingesetzt wird.

11. Verfahren nach Anspruch 10, **dadurch gekennzeichnet, daß** die Partikel eine durchschnittliche Größe von 0,3 bis 2,5 mm aufweisen.

## Claims

1. A process for the production of an oral, retarded release preparation from tramadol or a physiologically acceptable salt of tramadol with a storage-stable release profile of the active substance, **characterised in that** the active substance preparation is coated with an aqueous ethylcellulose dispersion which contains at least one physiologically acceptable, lipophilic diester of an aliphatic or aromatic C₆-C₄₀ dicarboxylic acid and an aliphatic C₁-C₈ alcohol as plasticiser, and the coating is dried at product temperatures of 35°C to 80°C only during coating without any curing after the drying.

2. A process according to claim 1, **characterised in that** the plasticiser used is a diester of an aliphatic or aromatic C₆-C₃₀, preferably C₁₀-C₁₆, dicarboxylic acid and an aliphatic C₂-C₆, preferably C₂-C₅, alcohol.

3. A process according to claim 2, **characterised in that** dibutyl phthalate, diethyl phthalate, dibutyl sebacate or diethyl sebacate, preferably dibutyl sebacate, is used as the plasticiser.

4. A process according to any one of claims 1 to 3, **characterised in that** the plasticiser is used in quantities of 5 to 50 wt.%, preferably 10 to 40 wt.%, particularly preferably 10 to 30 wt.%, relative to ethylcellulose.

5. A process according to any one of claims 1 to 4, **characterised in that** the concentration of ethylcellulose in the aqueous dispersion is from 3 wt.% to 35 wt.%, preferably 10 to 30 wt.%.

6. A process according to any one of claims 1 to 5, **characterised in that** drying of the retarding coating proceeds is carried out at temperatures of 40 to 80°C, preferably 50 to 70°C.

7. A process according to any one of claims 1 to 6, **characterised in that** a protective coating of watersoluble polymers, preferably hydroxypropylmethylcellulose or hydroxypropylcellulose and talcum is applied under the retarding coating.

8. A process according to claim 7, **characterised in that** the weight increase due to the protective coating amounts to 1 to 10 wt.%, preferably 2.5 to 5 wt.%.

9. A process according to any one of claims 1 to 8, **characterised in that** the physiologically acceptable salt of tramadol is tramadol hydrochloride.

10. A process according to any one of claims 1 to 9, **characterised in that** the active substance preparation is used as a multiparticulate active substance preparation, preferably as tablets, microtablets, granules, pellets or crystals.

11. A process according to claim 10, **characterised in that** the particles have an average size of 0.3 to 2.5 mm.

## Revendications

1. Procédé pour la fabrication d'une préparation orale, à effet retard, de tramadol ou d'un sel de tramadol, physiologiquement compatible et présentant un profil de libération de principe actif, stable au stockage, **caractérisé en ce que** l'on enrobe la préparation contenant le principe actif avec une dispersion aqueuse d'éthylcellulose qui renferme en tant que plastifiant au moins un diester lipophile, physiologiquement compatible, issu d'un acide dicarboxylique aliphatique ou aromatique en C₆-C₄₀ et d'un alcool aliphatique en C₁-C₈ et **en ce qu'**on ne sèche l'enrobage à des températures de 35 °C à 80 °C que pendant la phase d'enrobage, sans traitement de stabilisation par durcissement subséquent au séchage.

2. Procédé selon la revendication 1, **caractérisé en ce qu'**en tant que plastifiant, on emploie un diester issu d'un acide dicarboxylique aliphatique ou aromatique en C₆-C₃₀, de préférence en C₁₀-C₁₆ et d'un alcool aliphatique en C₂-C₆, de préférence en C₂-C₅.

3. Procédé selon la revendication 2, **caractérisé en ce qu'**en tant que plastifiant, on emploie le dibutylphtalate, le diéthylphtalate, le dibutylsébaçate ou le diéthylsébaçate, de préférence le dibutylsébaçate.

4. Procédé selon l'une des revendications 1 à 3, **caractérisé en ce que** le plastifiant est employé en quantités allant de 5 à 50 % en poids, de préférence de 10 à 40 % en poids, et selon une préférence particulière de 10 à 30 % en poids, par rapport à l'éthylcellulose.

5. Procédé selon l'une des revendications 1 à 4, **caractérisé en ce que** la concentration d'éthylcellulose dans la dispersion aqueuse se situe entre 3 % en poids et 35 % en poids, de préférence entre 10 et 30 % en poids.

6. Procédé selon l'une des revendications 1 à 5, **caractérisé en ce que** le séchage de l'enrobage à effet retard intervient à des températures de 40 à 80 °C, de préférence de 50 à 70 °C.

7. Procédé selon l'une des revendications 1 à 6, **caractérisé en ce que** sous l'enrobage à effet retard est déposé un enrobage protecteur à base de polymères solubles dans l'eau, de préférence l'hydroxypropylméthylcellulose ou l'hydroxypropylcellulose et le talc.

8. Procédé selon la revendication 7, **caractérisé en ce que** l'augmentation de poids provoquée par l'enrobage protecteur se situe entre 1 et 10 % en poids, de préférence entre 2,5 et 5 % en poids.

9. Procédé selon l'une des revendications 1 à 8, **caractérisé en ce que** le sel physiologiquement compatible de tramadol est le chlorure de tramadol.

10. Procédé selon l'une des revendications 1 à 9, **caractérisé en ce que** la préparation contenant le principe actif est mise en oeuvre en tant que préparation multi-particulaire du principe actif, de préférence sous forme de comprimés, de micro-comprimés, de granulés, de pastilles ou de cristaux.

11. Procédé selon la revendication 10, **caractérisé en ce que** les particules présentent une taille moyenne de 0,3 à 2,5 mm.
